# FASCICULE DE BREVET EUROPEEN

(11) **EP 2 124 833 B1**
(45) Date de publication et mention de la délivrance du brevet: **04.08.2010**
(21) Numéro de dépôt: 08799857.1
(22) Date de dépôt: 14.03.2008
(51) Int. Cl.: A61F 2/42, A61F 2/00

(54) **DISPOSITIF D'OSTEOSYNTHESE**
OSTEOSYNTHESEVORRICHTUNG
OSTEOSYNTHESIS DEVICE

(30) Priorité: 20.03.2007 FR 0702003
(43) Date de publication de la demande: 02.12.2009
(73) Titulaire: Memometal Technologies, 35170 Bruz (FR)
(72) Inventeur: PRANDI, Bernard, F-35700 Rennes (FR); BELLEMERE, Philippe, F-44000 Nantes (FR); AUGOYARD, Marc, F-69160 Tassin La Demi Lune (FR); PEYROT, Jacques, F-69160 Tassin La Demi Lune (FR); MEUSNIER, Tristan, 42100 Saint-etienne (FR)
(74) Mandataire: Benech, Frédéric
(86) Numéro de dépôt international: PCT/FR2008/050453
(87) Numéro de publication internationale: WO 2008/129214

(56) Documents cités:
- WO-A-2006/109004
- FR-A- 2 846 545
- US-A- 3 466 669
- US-A- 5 425 777

## Description

L'invention se rattache au secteur technique des implants orthopédiques, notamment pour arthrodèses et ostéosynthèses.

On rappelle qu'un implant d'ostéosynthèse doit servir à maintenir en place deux (ou plus) parties d'un même os fracturé ou coupé par une opération de chirurgie (ostéotomie), le temps nécessaire à la consolidation de cet os (typiquement 3 mois).

On rappelle qu'une arthrodèse est le blocage d'une articulation par voie chirurgicale pour souder deux os en un seul, au moyen d'un dispositif d'ostéosynthèse.

On rappelle que le but de toute ostéosynthèse et particulièrement dans le cas d'une arthrodèse est d'obtenir une très bonne stabilité aussi bien primaire que secondaire afin d'obtenir la consolidation dans les meilleures conditions, c'est-à-dire dans la position choisie par le chirurgien, en minimisant les problèmes de douleur postopératoire et les oedèmes, et en raccourcissant autant que faire se peut le temps de consolidation.

Pour ce résultat, la stabilité de l'ostéosynthèse liée à l'implant est critique. De plus, l'implant doit également apporter et maintenir une légère compression sur les parties d'os à fusionner, ce qui facilite cette consolidation.

Différentes solutions techniques ont été proposées, pour réaliser une arthrodèse, notamment au niveau des extrémités (pied, main, poignet, cheville, ...).

On peut citer, par exemple, des agrafes basiques qui n'assurent pas un bon maintien pendant la consolidation, et des agrafes à mémoire de forme qui permettent de mettre en compression les deux parties d'os à consolider, ce qui correspond au but recherché.
Toutefois, pour obtenir une stabilité satisfaisante, il est nécessaire de mettre deux, voire trois agrafes, dans des plans différents. Il en résulte un encombrement important, ce qui limite les applications, notamment sur des petits os (par exemple au niveau des doigts ou des orteils).

Il est également courant d'utiliser des plaques et vis extramédullaires ou extra osseuses, qui nécessitent également un encombrement relativement important et ne peuvent être utilisées sur les phalanges terminales des doigts (arthrodèse interphalangienne distale par exemple). Par ailleurs la stabilité à moyen terme de ces systèmes n'est pas toujours au rendez-vous (débricolage du montage).

Certains types de vis peuvent être utilisés en intramédullaire, mais dans ce cas, la voie d'abord nécessite un abord pulpaire ce qui peut générer des complications graves (sepsis, ...) et un inconfort pour le patient.

On peut également utiliser des broches qui présentent un encombrement réduit. Toutefois la stabilité obtenue n'est pas satisfaisante (problèmes de migration) et il est généralement nécessaire de procéder au retrait après consolidation. De plus avec de tels dispositifs, le patient ne peut immerger le doigt ou l'orteil traité puisque la broche est en général débouchante à l'extérieur de la peau.

Il existe pour des os longs (tibia, fémur, humérus, ....) des systèmes d'ostéosynthèse intramédullaires. On connaît par exemple les clous centromédullaires vérouillables. Outre que la technique de vérouillage est difficile, ils ne peuvent être miniaturisés pour la chirurgie des extrémités (main et pied).

Enfin il existe des dispositifs intramédullaires à mémoire de forme permettant de résoudre une partie de la problématique de l'arthrodèse ou de l'ostéosynthèse des petits fragments : par exemple les solutions décrites dans le brevet 2 846 545 ou le brevet 2 884 406.

Le brevet 2 846 545 décrit un dispositif en H qui s'ouvre dans le corps en forme de X, grâce à l'utilisation de la mémoire de forme réglée vers 37°C, chaque patte étant implantée dans un trou calibré.

En pratique un tel système ne permet pas une introduction correcte dans l'os. En effet, la réalisation de 2 trous parallèles est très difficile dans une phalange du fait de la taille et surtout les pattes parallèles ont tendance à s'ouvrir naturellement lors de l'introduction et ont plutôt ce faisant un effet de distraction des deux fragments que de mise en compression.

Par ailleurs, l'utilisation de la mémoire de forme est très limitative par les contraintes qu'elle génère pour les chirurgiens, notamment de gestion de la température : il faut impacter l'implant dans l'os dans leur phase froide fermée avant qu'ils s'ouvrent à la température du bloc. Ceci nécessite de mettre l'implant dans un support, de le stocker au froid et d'aller très vite pour l'implanter.

Enfin les pattes étant droites conduisent dans leur forme ouverte à créer un appui ponctuel à leur extrémité qui n'apporte pas une bonne tenue et peut abîmer l'os.

Le brevet 2 884 406 présente un système qui permet une introduction facilité soit par la forme (oeil), soit par un support ou une pince qui maintient fermées les pattes de l'implant pendant l'introduction.

Néanmoins, ces systèmes ne fonctionnent pas de manière très fiables, car ils ne définissent pas les critères optimaux permettant une bonne introduction dans l'os et un bon ancrage : les zones d'ancrage ont toujours tendance à s'ouvrir trop tôt, ce qui bloque l'introduction

L'invention s'est fixée pour but de remédier à tous ces inconvénients d'une manière simple, fiable, efficace et rationnelle.

Le problème que se propose de résoudre l'invention est de définir les critères de réussite pour un implant intramédullaire, facile à poser et efficace pour générer une stabilité primaire et secondaire du foyer d'ostéosynthèse ou d'arthrodèse grâce à sa rigidité et à sa composante de compression.

L'implant de l'invention est caractérisé par le fait qu'il comprend deux zones d'ancrage osseux de part et d'autre d'une zone rigide de stabilité, résistante au cisaillement, ces deux zones d'ancrage présentant à leur base une possibilité de réformation important (notamment par élasticité) et un design tels qu'elles peuvent adopter une position fermée (notamment grâce à une pince adaptée serrée à leur base) pour une introduction facile dans un trou centromédullaire calibré (réalisé avec un instrument adéquat), et qu'elles présentent dans le site osseux de part cette configuration particulière la possibilité de réaliser l'impaction finale sans effet distracteur sur l'os et présente une expansion suffisante pour assurer une bonne accroche dans l'os.

Les zones d'ancrage peuvent se déformer à leur base par élasticité, superélasticité ou mémoire de forme et sont typiquement constituées par des branches ou pattes, éventuellement reliées (forme en olive ou ballon de rugby). Ces branches présentent dans la forme ouverte un angle positif vers l'extérieur à leur base et sont courbées vers l'intérieur vers leur extrémité tandis quand forme fermée, l'angle à la base est inversé, c'est-à-dire négatif ou tourné vers l'intérieur ce qui permet d'avoir la largeur au niveau des extrémités (côté impaction) plus fine que leur base afin de ne pas risquer de s'impacter dans l'os et de bloquer la pénétration de l'implant.

L'invention trouve une application particulièrement avantageuse, qui ne saurait toutefois être considérée comme limitative, pour la réalisation des arthrodèses au niveau des phalanges des doigts et des orteils, notamment pour les articulations interphalangiennes proximales et distales dans la main et le pied.

L'ensemble est implantable par voie dorsale (ou éventuellement latérales ou palmaire/plantaire), mais sans abord pulpaire, ce qui minimise les risques d'infection et améliore le confort patient.

Pour tenir compte des caractéristiques anatomiques, les zones d'ancrages sont reliées à la zone médiane servant de résistance (notamment au cisaillement) au niveau du foyer d'ostéosynthèse par des zones de liaisons plus ou moins longues, et la zone centrale peut présenter une angulation pour s'adapter aux caractéristiques de l'arthrodèse recherchée.

Le matériau constitutif de l'implant objet de l'invention doit permettre une certaine ouverture minimale des zones d'ancrage une fois l'implant dans le corps. Il peut donc s'agir de n'importe quel matériau implantable suffisamment élastique tel que de l'acier inoxydable, du titane, un matériau biorésorbable tel que l'acide polylactide PLLA ...
Préférentiellement, l'implant objet de l'invention est réalisé à partir d'un matériau à mémoire de forme utilisé pour sa propriété de superélasticité (ou élasticité liée à la transformation de phase austénite martensite sous contrainte) qui présente la plus grande plage d'élasticité connue (jusqu'à 8% en allongement élastique équivalent en traction pour un implant en Nitinol, alliage de Nickel - Titane comprenant environ 55,5 à 56% en poids de Nickel, le reste en titane).

L'utilisation d'un matériau présentant une mémoire de forme thermique vers 37°C est également possible.

L'invention est exposée ci-après plus en détail à l'aide des figures et dessins annexés dans lesquels :
- La figure 1 présente un exemple d'implant selon l'invention dans sa position totalement ouverte, en 3 dimensions.
- La figure 2 présente ce même exemple dans sa position fermée d'introduction, dans son plan principal,
- La figure 3 présente un exemple d'implant selon l'invention dans sa position totalement ouverte, dans son plan principal.
- La figure 4 présente un exemple d'implant coudé selon l'invention dans sa position totalement ouverte
- La figure 5 (5a à 5f) présente la séquence d'implantation : fermé, introduit à moitié toujours contraint, introduit complètement d'un côté (libre dans l'os), et la même séquence de l'autre côté.

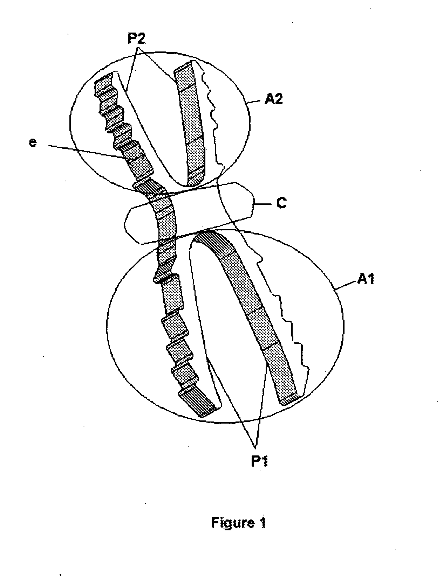

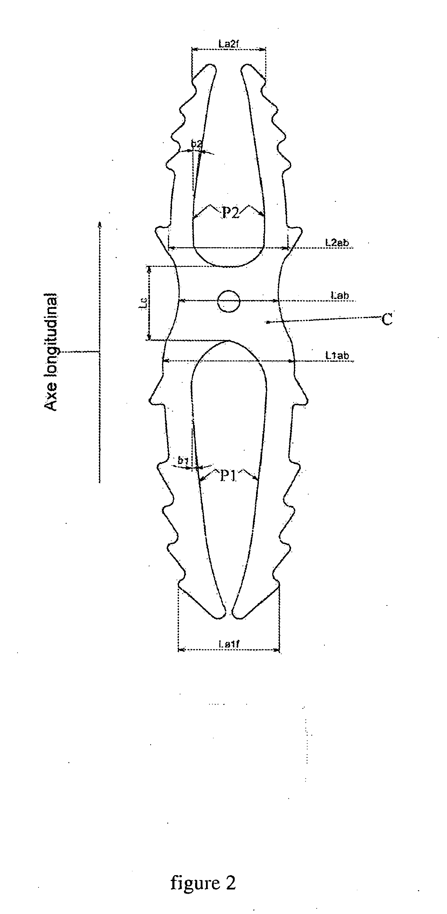

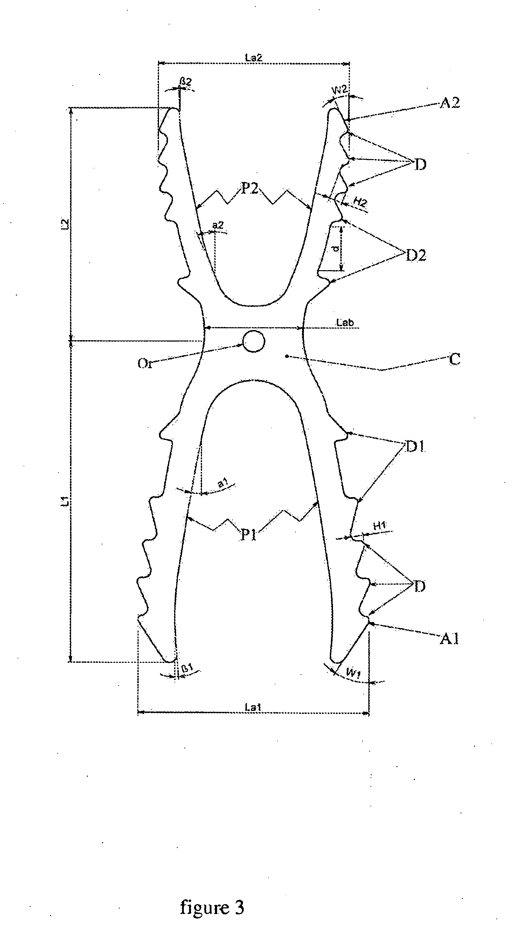

L'implant se présente sous la forme de 2 zones d'ancrages (A1) et (A2) reliées par une zone centrale (C) (figure 1) et éventuellement des zones de liaison intermédiaires telles que en position fermée, la forme soit sensiblement inscrite dans un rectangle très allongé (figure 2), et en forme ouverte corresponde plus à une forme en X plus large du fait de l'ouverture des zones d'ancrage (figure 3).

Les zones d'ancrages (A1), respectivement (A2) sont réalisées en général chacune par 2 pattes (P1), respectivement (P2) de longueur (L1), respectivement (L2) (figure 3).

La section transverse de l'implant est adaptée aux sites d'implantation, mais préférentiellement méplate afin d'avoir une bonne résistance mécanique et un encombrement réduit (typiquement l'épaisseur (e) est de l'ordre de 1 à 2 mm) (figure 1).

La figure 2 décrit la position fermée avec les différentes largeurs de l'implant : (Lab) est la largeur de la zone centrale (C), (L1ab) et (L2ab) sont les largeurs à la base des zones d'ancrage respectivement (A1) et (A2). Ces 3 largeurs peuvent être égales ou légèrement différentes pour s'adapter au site osseux. Typiquement les largeurs sont de l'ordre de 2 à 5 fois l'épaisseur (soit 2 à 10 mm). Ces dimensions sont bien adaptées aux différentes indications de la main et du pied mais ne sont pas limitatives puisqu'elles dépendent du site osseux du patient opéré.

Les zones d'ancrages (A1), (A2) sont aptes à s'écarter par effet élastique ou par effet mémoire de forme à leur base, afin que la largeur maximale en position ouverte aux extrémités (La1) et (La2) (figure 3) soit au moins égale à la largeur de la base de la même zone d'ancrage en position fermée augmentée de 50% minimum, ou augmentée de 1,5 mm minimum. C'est-à-dire que La1 > L1ab + 50% ou La1 > L1ab + 1,5 mm et que La2 > L2ab + 50% ou La2 > L2ab + 1,5 mm.

Ce critère d'ouverture est nécessaire afin d'avoir suffisamment de tenue dans l'os.

Ainsi que le décrit la figure 3, les pattes (P1), (P2) sont sensiblement droites à leur base (sur environ 1/3 à la moitié de leur longueur) puis de formes arrondies vers l'intérieur à leur extrémité (sur environ 1/3 à la moitié de leur longueur). En position ouverte la partie droite des pattes (P1), respectivement (P2) présente un angle (a1), respectivement (a2) avec l'axe de l'implant tourné vers l'extérieur (figure 3), positif, tandis qu'en position fermée, cet angle devient l'angle (b1), respectivement (b2) négatif tourné vers l'intérieur (figure 2). La partie supérieure (vers l'extrémité) des pattes ne subit quasiment pas de déformation particulière entre les 2 formes ouverte et fermée.
Cette disposition géométrique particulière permet qu'en position fermée les pattes se touchent quasiment à l'extrémité (figure 2), et que la largeur au niveau de l'extrémité en position fermée (La1f), respectivement (La2f) soit inférieure à la largeur de la base (L1ab), respectivement (L2ab), ce qui permet une introduction facile sans distraction du fragment osseux distal et permet également d'obtenir le mouvement ouvert/fermée par une déformation localisée à la base des pattes, c'est-à-dire en laissant libre la zone distale pour pouvoir introduire cette zone dans l'os.

Afin d'obtenir à la fois une introduction aisée et un mouvement suffisant d'ouverture, les angles (a1), (a2) sont préférentiellement compris entre +5° et +25° et les angles (b1), (b2) entre 0° et-15°.

Préférentiellement la largeur des extrémités des zones d'ancrage en position fermée (La1f), respectivement (La2f) est plus faible que la largeur de la base desdites zones (L1ab), respectivement (L2ab) diminuée de 20% : La1f< L1ab-20% et La2f< L2ab-20%.

Les pattes ou les zones d'ancrage sont ainsi « articulées » à leur base, et peuvent donc être maintenues en position fermées par un support ou mieux une pince, positionnée à un endroit adéquat défini notamment dans le cas d'un matériau élastique (pour un matériau à mémoire de forme, cela n'est pas forcément nécessaire puisque la forme n'évolue pas tant que la température d'activation n'est pas atteinte), cette pince ne couvrant pas plus de la moitié de la longueur des pattes, ce qui permet une introduction de l'implant d'au moins la moitié dans son logement.

La tangente côté interne en extrémité des pattes (P1), respectivement (P2) en position ouverte fait un angle (β1), respectivement (β2) avec l'axe longitudinal de l'implant proche de 0°, afin d'avoir une bonne surface de contact osseux sur toute la longueur de la patte en position ouverte et éviter que seules les extrémités touchent l'os (figure 3).

Dans le site d'implantation, à la température du corps, l'implant peut encore être en position fermée, ou pattes parallèles ou semi-ouvert, afin que l'effort exercé par l'ouverture des pattes se transmette au niveau de l'os et assure un bon ancrage.

Cette disposition « en olive » des pattes, associée à une « articulation » de la base et associée à une introduction minimale de la moitié de leur longueur permet de finir l'impaction, une fois la pince enlevée.

Afin de garantir un bon fonctionnement, l'élasticité ou la mémoire de la pièce doit permettre de passer de la forme fermée (typiquement largeur 2 à 4 mm selon la taille du site) à une forme ouverte avec un mouvement significatif (+1,5 à + 3 mm environ).
De même la force d'expansion des pattes (ou de gonflement de l'olive) doit être significative : typiquement 1 à 3 kg pour une arthrodèse des extrémités (force mesurée à 37°C en position d'introduction bloquée), sans être excessive : il importe que les pattes ne puissent pas s'ouvrir totalement et que l'os résiste afin d'avoir réellement une force de maintien.

Les pattes (P1), (P2) ou ailettes peuvent présenter une surface rugueuse ou mieux des crans (D) (figure 3) sur leurs surfaces externes destinées à s'impacter dans l'os spongieux et à exercer un bon ancrage. La hauteur typique de ces crans (H1), (H2) est de l'ordre de 0,5 mm.
L'ouverture des pattes doit être au minimum de 1,5 fois cette hauteur afin d'assurer une impaction réelle des crans dans l'os, soit 1,5 mm.

Les pattes (P1), (P2) peuvent également présenter une surface recouverte d'un revêtement ostéointégrateur telle que l'hydroxyhapatite (HAP) destinée à faciliter l'ancrage.

Afin de faciliter l'introduction dans l'os, les extrémités des pattes (P1), resp. (P2) sont biseautées avec un angle vers l'intérieur par rapport à l'axe longitudinal de l'implant (W1), resp. (W2) (figure 3). Cet angle est typiquement compris entre -20° et -40°.

Par essai sur cadavres frais, et expérience, il a été déterminé un niveau optimal de la force avec un minimum permettant d'ancrer les crans dans l'os spongieux et une force maximale pour être sur de ne pas détériorer le site d'implantation

Après tests et expérience, il a été trouvé une zone idéale de maximum 20% de la limite élastique de l'os mesurée en forme fermée bloquée à 37°C , ce qui compte tenu des dimensions de l'implant conduit à des valeurs maximales de l'ordre de 3 kg, et la nécessité d'un abaissement rapide dès que l'ancrage est obtenu, soit une force divisée par 2 en position semi-ouverte (une force de 0,5 à 1,5 kg permet un bon maintien).
En effet si la force d'ouverture est supérieur à environ 3 kg, l'introduction dans l'os devient beaucoup plus difficile, voir impossible dès 4 kg.
Enfin afin de garantir une non détérioration du site, il est nécessaire que la force devienne négligeable pour une ouverture quasi-complète.
Ces valeurs sont indicatives et dépendent du site d'arthrodèse et de la qualité osseuse.

Sur une version de l'invention, des crans (D1), resp.(D2) côté externe aux pattes (P1), resp. (P2) permettent de positionner une pince de serrage et d'introduction à la base des pattes (P1), (P2) (figure 3). Ces crans sont asymétriques par paire de pattes et leur écartement (d) est le même sur les pattes (P1) que sur les pattes (P2).

La zone centrale (C) doit avoir une longueur minimale (Lc) égale à la longueur (d) entre les crans (D1), respectivement (D2), afin que même en cas de mouvement de l'implant lors de l'impaction finale cette zone (C) resté dans le foyer d'arthrodèse et assure sa fonction de résistance.
Dans une version de l'invention, il est prévu un orifice (Or) dans cette zone centrale permettant de positionner une broche de maintien pour éviter une éventuelle migration de l'implant au moment de l'impaction finale.

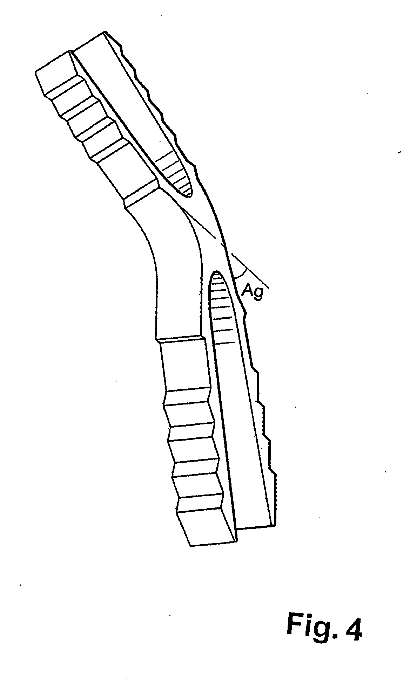

Ainsi que le montre la figure 4, cette zone centrale peut être angulée d'un angle (Ag) défini entre les 2 plans principaux formés par les pattes (P1) d'une part et (P2) d'autre part pour s'adapter aux contraintes chirurgicales de réglage de la position de l'arthrodèse. Dans la majorité des cas, l'angle (Ag) est fixé entre 0° (position plate typiquement pour un index) et 30°(typiquement pour un 5^{e} doigt)

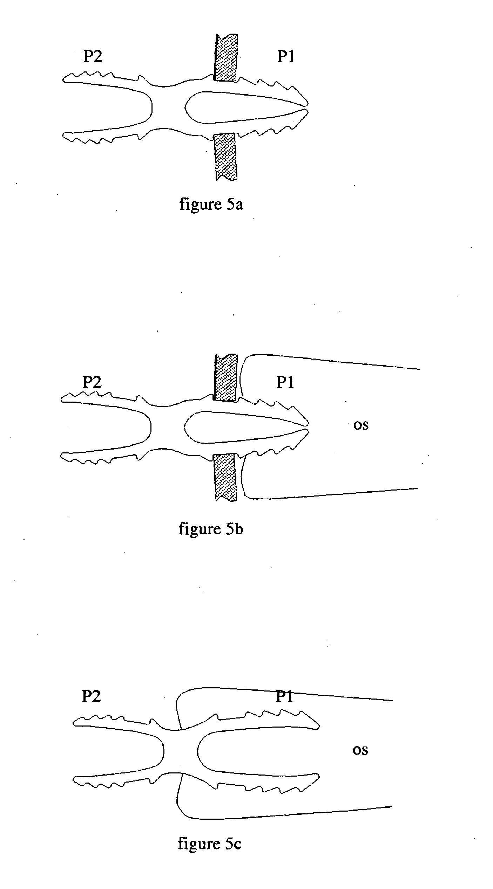

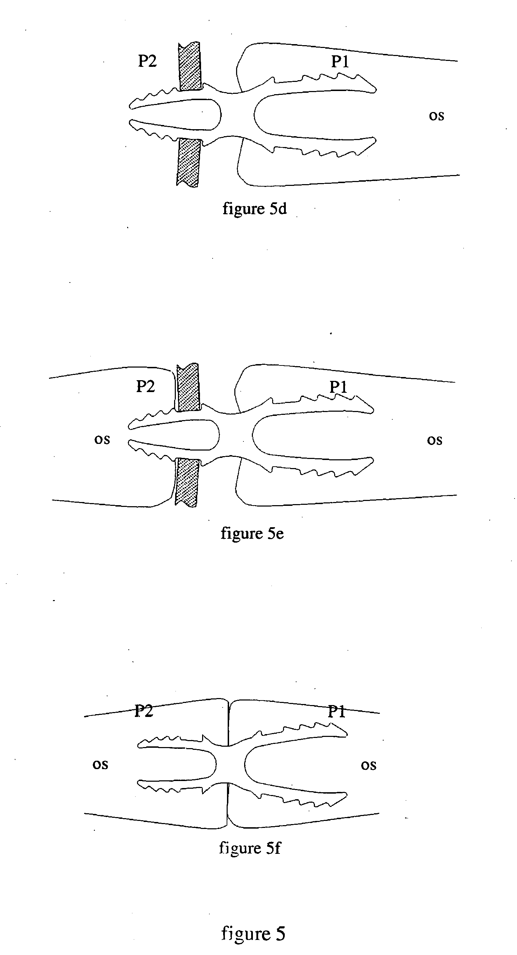

A titre d'exemple une technique opératoire de l'implantation du dispositif de l'invention pour le cas d'un implant élastique ou superélastique est la suivante telle que décrit sur la figure 5 :
Abord par voie dorsale
Résection des cartilages et ostéophytes
Perçage centromédullaire à l'aide d'un instrument adapté permettant de faire un trou calibré rectangle de largeur sensiblement (L1ab) ou (L2ab) et d'épaisseur sensiblement e (râpe adaptée)
Fermeture à la pince côté P1 (figure 5a)
Introduction implant côté P1 à la moitié minimum (figure 5b)
Enlèvement pince
Introduction totale côté P1 (figure 5c)
Fermeture à la pince côté P2 (figure 5d)
Mise en place de l'os côté P2 sur l'implant côté P2 à la moitié environ (figure 5^{e}) Enlèvement de la pince
Impaction manuelle de l'os côté P2 sur l'os P1 (figure 5f)

Dans une forme de réalisation particulière, destinée à l'arthrodèse de l'InterPhalangienne Distale (main), l'implant est réalisé dans un alliage nitinol superélastique (nickel-titane dans la proportion 55,8% poids Nickel et 44,2% titane)

La section de la zone centrale (C) est de Lab x e = 2,8 x 1,2 mm et les pattes sont dissymétriques pour s'adapter au mieux aux formes de l'os, minimiser la section métallique implantée et permettre une expansion suffisante pour un bon ancrage. La longueur des pattes est de L2=6,5 mm en distale (P2) et L1=9 mm en proximale (P1)
La longueur de la zone centrale (C) est de 3 mm, ce qui autorise un petit décalage lors de la fermeture, sans nuire à la résistance au cisaillement.
Pour s'adapter au choix du chirurgien, cette zone centrale peut être coudée (typiquement plate ou 15° ou 25°)

En position fermée, la largeur de la base proximale Ll ab de 3,8 mm et de la base distale L2ab est de 3,0 mm.

L'ouverture des pattes (P1), respectivement (P2) est de 2,5 mm, respectivement 2,2 mm, c'est-à-dire que La1 vaut 6,3 mm et La2 vaut 5,2 mm.
En position ouverte l'angle à la base des pattes est de a1 =10° et a2=22°
La portion droite est d'environ 45% de la longueur totale
La courbure de l'extrémité distale des pattes est calculée pour que l'angle de la tangente à l'extrémité soit de β1= -5° et β2= -3°

En position fermée, l'angle à la base des pattes est bl=-4°, b2= -2°
Et la largeur à l'extrémité est La1f = 2,5 mm et La2f = 2,1 mm

Dans une forme de réalisation de l'invention, des crans de hauteur 0,5 mm sont réparties sur les pattes (1 cran tous les 0,8 mm environ)

L'angle d'incidence de l'extrémité des pattes (crans compris) est de w1 = 33° et w2 = 24°, ce qui permet une introduction aisée sans effet distracteur entre les 2 morceaux d'os à ostéosynthéser

Le dessin arrondi des zones d'ancrage permet d'obtenir en forme ouverte une surface de contact maximisée sur toute la longueur, avec un effet d'impaction dans l'os spongieux, et donc un effet de tassement du spongieux.

Dans un autre exemple, plus adapté à une arthrodèse au niveau du pouce, les dimensions sont plutôt les suivantes :
Largeurs fermées : L1ab = 6,5 mm, L2ab = 5 mm, avec une ouverture de 3 à 4mm environ pour obtenir : La1 = 11 mm et La2 = 8 mm et L1=13 mm et L2=9mm

## Revendications

1. Dispositif d'ostéosynthèse et d'arthrodèse intramédullaire pour la chirurgie orthopédique notamment de la main et du pied, de section principale sensiblement méplate, de forme première en H constituée de 2 zones d'ancrage (A1), (A2) comprenant chacune un jeu de pattes (P1), (P2) sensiblement parallèles reliés par une zone centrale (C ), forme en H dite forme fermée ou forme d'introduction sensiblement inscrite dans un rectangle allongé, et une forme ouverte dite implantée en X de largeur supérieure, **caractérisée en ce que** :
- en forme ouverte les pattes (P1), (P2) forment à leur base sur le 1^{er} tiers de leur longueur un angle par rapport à l'axe de l'implant (a1), (a2) vers l'extérieur compris entre 5° et 25°, et sont recourbées vers l'intérieur sur le dernier tiers de leur longueur (vers leur extrémité), pour que la tangente côté interne à l'extrémité forme un angle (β1), (β2) avec l'axe longitudinal de l'implant voisin de 0°,
- en forme fermée les pattes (P1), (P2) forment à leur base sur le 1^{er} tiers de leur longueur un angle (b1), (b2) vers l'intérieur compris entre -0° et -15°, sans modification notables sur le dernier tiers de leur longueur, pour que la largeur extérieure aux extrémités en forme fermée (La1f), respectivement (La2f) et sur environ la moitié de la longueur des pattes côté extrémités soit inférieure à la largeur de la base (L1ab), respectivement (L2ab) de ces zones d'ancrage (A1), (A2),
- le passage de la forme fermée à la forme ouverte se fait préférentiellement par la déformation de la base des zones d'ancrage (A1), (A2).

2. Dispositif selon la revendication 1 **caractérisé en ce que** la largeur aux extrémités de la forme ouverte (La1), respectivement (La2) est au moins égale à la largeur d'introduction fermée (L1ab), respectivement (L2ab) plus 50%, ou à la largeur d'introduction fermée (L1ab), respectivement (L2ab) + 1,5 mm.
C'est-à-dire La1 > L1ab + 50% ou La1 > L1ab + 1,5 mm et La2 > L2ab + 50% ou La2 > L2ab + 1,5 mm.

3. Dispositif selon l'une quelconque des revendications 1 à 2 **caractérisé en ce que** la largeur aux extrémités de la forme fermée (La1f), respectivement (La2f) soit inférieure à la largeur en forme fermée de la base (L1ab) de la zone d'ancrage (A1), respectivement (L2ab) de la zone d'ancrage (A2) diminuée de 20% : La1f< L1ab-20% et La2f< L2ab-20%.

4. Dispositif selon l'une quelconque des revendications 1 à 3 **caractérisé en ce que** les extrémités de la zone à introduire sont biseautées vers l'intérieur pour faciliter l'introduction, en présentant un angle (W1), (W2) en position ouverte par rapport à l'axe de l'implant compris entre -20 et -40° vers l'intérieur.

5. Dispositif selon l'une quelconque des revendications 1 à 4 **caractérisé en ce que** les 2 zones d'ancrage présentent des crans (D) sur la surface externe des pattes (P1), (P2) ou une surface rugueuse destinés à faciliter l'ancrage.

6. Dispositif selon l'une quelconque des revendications 1 à 5 **caractérisé en ce que** les 2 zones d'ancrage sont reliées par une zone centrale (C) rigide apte à supporter les contraintes de cisaillement au niveau du foyer d'arthrodèse.

7. Dispositif selon l'une quelconque des revendications 1 à 6 **caractérisé en ce que** la zone centrale (C) présente une angulation (Ag) entre les 2 zones d'ancrage (A1), (A2) permettant un réglage de la position osseuse de l'arthrodèse entre 0 et 30°.

8. Dispositif selon l'une quelconque des revendications 1 à 7 **caractérisé en ce qu'**il est réalisé dans un matériau implantable, notamment en nitinol (nickel titane à mémoire de forme ou superélastique), en titane élastique ou en un matériau résorbable tel que l'acide polylactide PLLA, de sorte que le passage de la forme d'introduction fermée à la forme ouverte se fasse par élasticité, superélasticité ou mémoire de forme.

9. Dispositif selon l'une quelconque des revendications 1 à 8 **caractérisé en ce que** il existe dans la moitié inférieure des zones d'ancrage une zone ou un logement de longueur (d) matérialisé par exemple par des crans externes (D1), (D2) permettant de positionner un instrument adéquat de fermeture et de maintien en position fermée.

10. Dispositif selon l'une quelconque des revendications 1 à 9 **caractérisé en ce que** la longueur (Lc) de la zone centrale (C) est au moins égale à la longueur (d) du logement matérialisé par les crans (D1), (D2).

11. Dispositif selon l'une quelconque des revendications 1 à 10 **caractérisé en ce que** il existe un orifice (Or) dans la zone centrale (C) perpendiculaire à la surface permettant de positionner un instrument ou une broche de maintien.

## Claims

1. Intramedullary osteosynthesis and arthrodesis device for orthopaedic surgery, in particular of the hand and the foot, having a substantially flat main section, with a primary H form consisting of 2 anchoring zones (A1), (A2) each comprising a set of substantially parallel legs (P1), (P2) connected by a central zone (C), in an H form known as the closed form or introduction form substantially inscribed in an elongated rectangle, and an open X form known as implanted with a larger width, **characterised in that**:
- in the open form, the legs (P1), (P2) form at their base, over the 1^{st} third of their length, an outward angle of between 5° and 25° relative to the axis of the implant (a1), (a2) and are curved towards the interior over the last third of their length (toward their end), so that the inside tangent at the end forms an angle (β1), (β2) of close to 0° with the longitudinal axis of the implant,
- in the closed form, the legs (P1), (P2) form at their base, over the 1^{st} third of their length, an inward angle (b1), (b2) towards the interior of between -0° and -15°, without any significant change over the last third of their length, so that the outside width at the ends in the closed form (La1f) and (La2f) respectively and over about half of the length of the legs on the end side is less than the width of the base (L1ah) and (L2ab) respectively of these anchoring zones (A1), (A2),
- the transition from the closed form to the open form occurs preferably by deformation of the base of the anchoring zones (A1), (A2).

2. Device according to claim 1, **characterised in that** the width at the ends in the open form (La1) and (La2) respectively is at least equal to the closed introduction width (L1ab) and (L2ab) respectively plus 50% or to the closed introduction width (L1ab) and (L2ab) respectively + 1.5 mm, i.e. La1 > L1ab + 50% or La1 > L1ab + 1.5 mm and La2 > L2ab + 50% or La2 > L2ab + 1.5 mm.

3. Device according to either one of claims 1 to 2, **characterised in that** the width at the ends in the closed form (La1f) and (La2f) respectively is less than the width in the closed form of the base (L1ab) of the anchoring zone (A1) and (L2ab) respectively of the anchoring zone (A2) less 20%: La1f < L1ab-20% and La2f < L2ab-20%,

4. Device according to any one of claims 1 to 3, **characterised in that** the ends of the zone to be introduced are bevelled towards the interior to facilitate introduction, displaying an angle (W1), (W2) in the open position relative to the axis of the implant of between -20° and -40° towards the interior.

5. Device according to any one of claims 1 to 4, **characterised in that** the 2 anchoring zones have notches (D) on the external surface of the legs (P1), (P2) or a rough surface intended to facilitate the anchoring.

6. Device according to any one of claims 1 to 5, **characterised in that** the 2 anchoring zones are connected by a rigid central zone (C) capable of withstanding shear stresses in the region of the focus of the arthrodesis.

7. Device according to any one of claims 1 to 6, **characterised in that** the central zone (C) has an angulation (Ag) between the 2 anchoring zones (A1), (A2) allowing an adjustment of the bone position of the arthrodesis between 0 and 30°.

8. Device according to any one of claims 1 to 7, **characterised in that** it is made from an implantable material, in particular of nitinol (shape memory or superelastic nickel titanium), of elastic titanium or of a resorbable material such as polylactic acid PLLA, so that the transition from the closed introduction state to the open state occurs by elasticity, superelasticity or shape memory.

9. Device according to any one of claims 1 to 8, **characterised in that** the lower half of the anchoring zones contains a zone or a recess of length (d) formed for example by external notches (D1), (D2) allowing the positioning of a suitable instrument for closure and for holding in the closed position.

10. Device according to any one of claims 1 to 9, **characterised in that** the length (Lc) of the central zone (C) is at least equal to the length (d) of the recess formed by the notches (D1), (D2).

11. Device according to any one of claims 1 to 10, **characterised in that** the central zone (C) contains an orifice (Or) perpendicular to the surface for positioning an instrument or a holding pin.

## Patentansprüche

1. Intramedulläre Osteosynthese- und Arthrodesevorrichtung für die orthopädische Chirurgie, insbesondere der Hand und des Fußes, mit einem im wesentlichen flachen Hauptabschnitt, mit einer ersten Form als H aufweisend zwei Verankerungszonen (A1), (A2), von denen jede einen Satz von im wesentlichen parallelen Schenkeln (P1), (P2) aufweist, die an eine Zentralzone (C) angebunden sind, wobei die Form als H als eine geschlossene Form oder Einführungsform im wesentlichen in einem länglichen Rechteck einbeschrieben ist, und wobei eine offene Form vorgesehen ist, implantiert als X mit einer größeren Breite,
**dadurch gekennzeichnet, dass**
- in der offenen Form die Schenkel (P1), (P2) an ihrer Basis auf einem ersten Drittel ihrer Länge einen nach außen geöffneten Winkel von zwischen 5° und 25° zu einer Achse der Implantation (a1), (a2) bilden, und auf einem äußersten Drittel der Länge (in Richtung zu ihrer Spitze) nach innen gekrümmt sind, so dass eine innere Tangente an der Spitze einen Winkel (β1), (β2) von nahezu 0° mit der Achse in Längsrichtung der Implantation bildet,
- in der geschlossenen Form die Schenkel (P1), (P2) an ihrer Basis auf dem ersten Drittel ihrer Länge einen nach innen geneigten Winkel (b1), (b2) von zwischen - 0° und - 15° ohne eine bemerkbare Änderung längs des äußersten. Drittels der Länge bilden, so dass eine äußere Breite an den Spitzen in der geschlossenen Form (La1f) beziehungsweise (La2f) und auf ungefähr der Hälfte der Länge der Schenkel auf der Seite der Spitzen kürzer ist als die Breite der Basis (L1 ab) beziehungsweise (L2ab) in den Verankerungszonen (A1), (A2), und
- der Übergang von der geschlossenen Form zu der offenen Form vorzugsweise durch eine Deformation der Basis der Verankerungszone (A1), (A2) erzielt wird.

2. Vorrichtung nach Anspruch 1,
**dadurch gekennzeichnet,**
**dass** die Breite an den Spitzen der offenen Form (La1) beziehungsweise (La2) zumindest gleich der Breite der geschlossenen Einführung (L1ab) beziehungsweise (L2ab) zuzüglich 50 % beträgt, oder der Breite der geschlossenen Einführung (L1ab) beziehungsweise (L2ab) zuzüglich 1,5 mm, das heißt
La1 >.L1ab + 50 % oder La1 > L1ab + 1,5 mm und L2a > L2ab + 50 % oder La2 > L2ab + 1,5 mm.

3. Vorrichtung nach einem der Ansprüche 1 oder 2,
**dadurch gekennzeichnet,**
**dass** die Breite an den Spitzen der geschlossenen Form (La1f) beziehungsweise (La2f) kleiner ist als die Breite in der geschlossenen Form an der Basis (L1ab) der Verankerungszone (A1) beziehungsweise an der Basis (L2ab) der Verankerungszone (A2) verringert um 20 %:
La1f < L1 ab - 20 % und La2f < L2ab - 20 %.

4. Vorrichtung nach einem der Ansprüche 1 bis 3.
**dadurch gekennzeichnet,**
**dass** die Spitzen der Zone beim Einführen zur Erleichterung der Einführung nach innen geneigt werden und einen Winkel (W1), (W2) darstellen, der in einer geöffneten Position bezüglich der Achse der Implantation zwischen -20° und -40° nach innen einschließt.

5. Vorrichtung nach einem der Ansprüche 1 bis 4,
**dadurch gekennzeichnet,**
**dass** die zwei Verankerungszonen Zacken (D) auf der äußeren Oberfläche der Schenkel (P1), (P2) oder eine raue Oberfläche für die leichtere Verankerung zeigen.

6. Vorrichtung nach einem der Ansprüche 1 bis 5,
**dadurch gekennzeichnet,**
**dass** die zwei Verankerungszonen über eine steife zentrale Zone (C) angebunden sind, die geeignet ist, Scherbelastungen in der Ebene des Bereiches der Arthrodese standzuhalten.

7. Vorrichtung nach einem der Ansprüche 1 bis 6,
**dadurch gekennzeichnet,**
**dass** die zentrale Zone (C) eine Winkelbestimmung (Ag) zwischen den zwei Verankerungszonen (A1), (A2) zur Verfügung stellt, die eine Regelung der Position der Knochen der Arthrodese zwischen 0° und 30° ermöglicht.

8. Vorrichtung nach einem der Ansprüche 1 bis 7,
**dadurch gekennzeichnet,**
**dass** sie mit einem implantierbaren Werkstoff ausgeführt ist, insbesondere aus Nitinol (Nickeltitan mit Formgedächtnis oder superelastisch), aus elastischem Titan oder aus einem resorbierbaren Werkstoff wie Polymilchsäure (PLS, PLLA), derart, dass der Übergang von der geschlossenen Einführungsform zu der offenen Form selbst durch Elastizität, durch Superelastizität oder durch Formgedächtnis geschieht.

9. Vorrichtung nach einem der Ansprüche 1 bis 8,
**dadurch gekennzeichnet,**
**dass** vorgesehen ist in der inneren Hälfte der Verankerungszone eine Zone oder ein Sitz der Länge (d) vorgesehen ist, verwirklicht zum Beispiel durch äußere Zacken (D1), (D2), die es erlauben, ein Instrument zu positionieren, geeignet zum Verschließen und zum Festhalten in der geschlossenen Position.

10. Vorrichtung nach einem der Ansprüche 1 bis 9,
**dadurch gekennzeichnet,**
**dass** die Länge (Lc) der zentralen Zone (C) wenigstens gleich der Länge (d) des Sitzes ist, der durch die Zacken (D1), (D2) verwirklicht ist.

11. Vorrichtung nach einem der Ansprüche 1 bis 10,
**dadurch gekennzeichnet,**
**dass** eine Öffnung (Or) in der zentralen Zone (C) senkrecht zu der Oberfläche vorgesehen ist, die es erlaubt, ein Instrument oder einen Haltestift zu positionieren.
